(19) Europäisches Patentamt
European Patent Office
Office européen des brevets

(11) **EP 2 067 495 A1**

(12) **EUROPEAN PATENT APPLICATION**
published in accordance with Art. 153(4) EPC

(43) Date of publication:
**10.06.2009 Bulletin 2009/24**

(51) Int Cl.:
***A61M 1/36*** *(2006.01)*     ***A61M 1/02*** *(2006.01)*

(21) Application number: **07828762.0**

(22) Date of filing: **28.09.2007**

(86) International application number:
**PCT/JP2007/069021**

(87) International publication number:
**WO 2008/038785 (03.04.2008 Gazette 2008/14)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **29.09.2006 JP 2006267462**

(71) Applicant: **Toray Industries, Inc.**
**Tokyo 103-8666 (JP)**

(72) Inventor: **SHIMAGAKI, Masaaki**
**Otsu-shi, Shiga 5208558 (JP)**

(74) Representative: **Webster, Jeremy Mark et al**
**Mewburn Ellis LLP**
**33 Gutter Lane**
**London**
**EC2V 8AS (GB)**

(54) **CELL-ADSORBING COLUMN**

(57) In light of such conventional technical problems, it is an object of the present invention to provide a cell adsorption column which has an adsorbent with a surface area where there is less concern about denaturation and differentiation induction of cells caused by a contact of the adsorbent with the cells, and whose adsorbent-filled volume is small, and which has a compact property for improving operability and reducing a burden on a patient.

The present invention provides a cell adsorption column filled with an adsorbent containing a fiber whose fiber diameter is 0.5 $\mu$m or more and 10 $\mu$m or less and having a surface area of 0.5 m$^2$ or more and less than 10 m$^2$, in which the adsorbent-filled volume is 100 mL or less.

FIG.1

**Description**

TECHNICAL FIELD

The present invention relates to a novel adsorption column, in particular to the adsorption column which is suitable for a blood treatment column used by circulating blood therein. The present invention relates to a cell adsorption column in which an adsorbent in a certain amount is incorporated and which can remove leukocytes in a sufficient amount and is excellent in compact property.

BACKGROUND ART

In recent years, various blood treatment columns have been studied, and for example, the columns for the purpose of removing leukocytes and granulocytes (JP Sho 60-193468 A, JP Hei 5-168706 A [Patent Documents 1 and 2]), the columns for the purpose of adsorbing cytokines (JP Hei-10-225515 A, JP Hei 12-237585 A and JP Hei 10-147518 A [Patent Documents 3, 4 and 7]) and the columns for the purpose of simultaneously adsorbing the leukocytes and toxins (JP 2002-113097 A [Patent Document 5]) have been developed. These typically have a filtration material or an adsorbent carrier therein to remove and adsorb target substances. Various materials in various shapes have been used as the filtration materials or the adsorbent carriers, and each of them has both advantages and disadvantages. For example, as to the leukocyte removal carrier composed of a polyester nonwoven fabric, which is disclosed in JP Sho 60-193468 A [Patent Document 1], the nonwoven fabric is made by mixing fibers having various fiber-diameters, thus the carrier is made to improve clogging of blood cells. However, a bulk density of the nonwoven fabric itself is high and the carrier can not control a removal of blood cells well, so still there is concern about a rise of pressure loss during the blood circulation.

As to the adsorbent carrier having a diameter of about 2 mm and composed of acetate cellulose beads ,which is disclosed in Patent Document 2, there is no concern about pressure loss, but it is not possible to increase a surface area for adsorption, and thus this is inefficient as the adsorbent carrier. Alternatively, a reduction of particle diameters leads to a rise of the pressure loss; therefore, this carrier is difficult to be employed.

It is disclosed in JP 2002-172163 A [Patent Document 6] that if a bulk density of an adsorbent carrier is too large, clogging occurs easily; on the other hand, if it is too small, morphological retention is deteriorated; therefore, it is important that a bulk density is 0.05 to 0.15 g/cm$^3$, and preferably is 0.10 to 0.15 g/cm$^3$ in order to apply to the carrier. However, the carrier having a range of 0.05 to 0.10 g/cm$^3$ is poor in shape stability, and the carrier having a range of 0.10 to 0.15 g/cm$^3$ is still impractical.

Among conventional cell adsorption columns, as a column having a high selectivity for cell removal, there is only a column of which an adsorbent-filled volume is such large that it excesses 150 mL or more (e.g., commercially available Adacolumn [registered trademark]). As a cell adsorption column having no selectivity to remove cells and mainly having a filtration function, there are columns which have a small volume for filling with the adsorbent (e.g., commercially available Sepacell [registered trademark]). However, in such columns, a surface area of the filled adsorbent exceeds 10 m$^2$, and then there is concern about influences such as denaturation and differentiation induction caused by a contact of the surface with cells.

Patent Document 1: JP Sho 60-193468 A
Patent Document 2: JP Hei 5-168706 A
Patent Document 3: JP Hei 10-225515 A
Patent Document 4: JP Hei 12-237585 A
Patent Document 5: JP 2002-113097 A
Patent Document 6: JP 2002-172163 A
Patent Document 7: JP Hei 10-147518 A

DISCLOSURE OF INVENTION

PROBLEM TO BE SOLVED BY THE INVENTION

In view of the foregoing problems in prior arts, it is an object of the present invention to provide a cell adsorption column which has a surface area of an adsorbent where there is less concern about denaturation and differentiation induction of cells caused by a contact of the adsorbent with the cells, and of which adsorbent-filled volume is small, and has a property of compactness for operability and for reducing a burden on a patient.
It is also another object of the present invention to provide a cell adsorption column which can remove not only cells present in blood, particularly activated leukocytes such as granulocytes and monocytes, and cancer cells, but also excessively existing cytokines, and which exhibits a decreased pressure loss. The blood referred to herein means a liquid including the cells and blood components.

MEANS FOR SOLVING PROBLEM

[1] A cell adsorption column filled with an adsorbent having a surface area of 0.5 $m^2$ or more and less than 10 $m^2$ and containing a fiber of which fiber diameter is 0.5 $\mu$m or more and 10 $\mu$m or less, wherein an adsorbent-filled volume is 100 mL or less.

[2] The cell adsorption column according to the above [1], wherein a bulk density of a portion composed of said fiber of which fiber diameter is 0.5 $\mu$m or more and 10 $\mu$m or less in said adsorbent is 0.01 $g/cm^3$ or more and 0.15 $g/cm^3$ or less.

[3] The cell adsorption column according to the above [1] or [2], wherein said fiber diameter is 3 $\mu$m or more and 10 $\mu$m or less.

[4] The cell adsorption column according to any one of the [1] to [3], wherein an amine residue is coupled to said adsorbent.

[5] The cell adsorption column according to any one of the above [1] to [4], which adsorbs a cytokine.

[6] The cell adsorption column according to the [5], wherein said cytokine is at least one selected from the group consisting of interleukin-1 (IL-1), interleukin-6 (IL-6), interleukin-8 (IL-8), interleukin-10 (IL-10), tumor necrosis factor-$\alpha$ (TNF-$\alpha$), transforming growth factor-$\beta$ (TGF-$\beta$), vascular endothelial growth factor (VEGF) and immunosuppressive acidic protein (IAP).

[7] The cell adsorption column according to any one of the above [1] to [6], which is used for circulating blood.

[8] The cell adsorption column according to the above [7], wherein the following formulae (1) and (2) are satisfied when the blood from a human healthy volunteer is passed through the column at a blood flow rate of 30 mL/minutes for one hour:

$$2c<a \qquad\qquad (1)$$

and

$$b>10^6 \qquad\qquad (2),$$

wherein,

a: the number of adsorbed granulocytes per unit weight of the adsorbent (number/g),
b: the number of adsorbed monocytes per unit weight of the adsorbent (number/g), and
c: the number of adsorbed lymphocytes per unit weight of the adsorbent (number/g).

[9] The cell adsorption column according to any one of the above [1] to [8], wherein a pressure loss is 150 mmHg or less between the front and the rear of said column at the time of 1500 mL of blood has been treated when said blood whose hematocrit value has been adjusted to 40% using saline is passed through said column at a rate of 30 mL/minute in an extracorporeal circulation test using a healthy dog.

[10] A method for adsorbing a cell, wherein said cell in a sample is adsorbed using the cell adsorption column according to any one of the above [1] to [9].

[11] The method for adsorbing the cell according to the above [10], wherein said sample is blood.

[12] The method for adsorbing the cell according to the above [10] or [11], wherein said cell is a leukocyte.

[13] A leukocyte reduction therapy using the cell adsorption column according to any one of the above [1] to [9].

[14] A therapy for ulcerative colitis, Crohn's disease or chronic rheumatoid arthritis using the cell adsorption column according to any one of the above [1] to [9].

EFFECT OF THE INVENTION

According to the present invention, it has become possible to provide a cell adsorption column which has the adsorbent with a surface area where there is less concern about denaturation and differentiation induction of cells caused by a contact with the cells, and which has the small scale of an adsorbent-filled volume, and which has the property of compactness for operability and for reducing a burden on patients. It has also become possible to provide a cell adsorption column which can remove not only cells present in blood, particularly activated leukocytes such as granulocytes and monocytes, and cancer cells but also the excessively existing cytokines, and which exhibits a decreased pressure loss.

BRIEF DESCRIPTION OF DRAWINGS

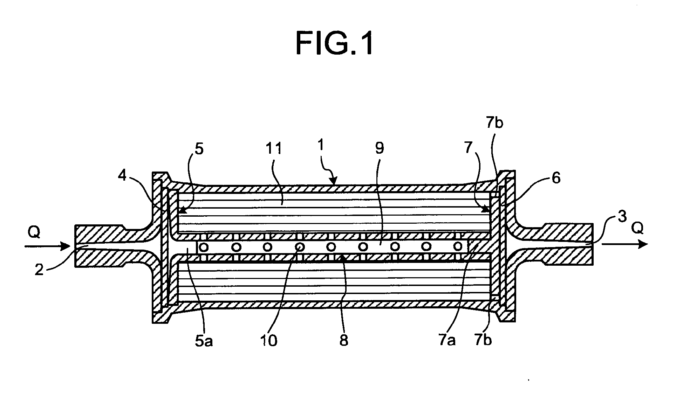

FIG. 1 is a vertical cross-sectional view exemplifying a cell adsorption column of the present invention.

EXPLANATIONS OF LETTERS OR NUMERALS

1    Container main body
2    Flow inlet
3    Flow outlet
4    Filter
5    Divider plate
5a   Opening of divider plate
6    Filter
7    Divider plate
7a   Support lug of divider plate
7b   Through hole of divider plate
8    Pipe
9    Flow path
10   Penetration hole
11   Adsorbent
Q    Flow of blood

BEST MODES FOR CARRYING OUT THE INVENTION

Cell adsorption by the cell adsorption column of the present invention means the state that the cells (granulocytes, monocytes, lymphocytes, other blood cells, cancer cells and the like) present in blood which have flowed in the column do not flow out from the column. And also, it includes a phenomenon based on a filtration effect in the present invention. That is, use of "cell adsorption" means a use for adsorbing cells from a body fluid such as blood in order to treat human bodies or prevent diseases.

It is desirable that the cell adsorption column of the present invention satisfies the following formulae (1) and (2) regarding the number of leukocytes adsorbed from blood when the blood from a healthy donor is circulated in the column for one hour.

$$2c < a \qquad (1)$$

$$b > 10^6 \qquad (2)$$

In the above formulae (1) and (2), a: the number of adsorbed granulocytes per unit weight of the adsorbent (number/g), b: the number of adsorbed monocytes per unit weight of the adsorbent (number/g), and c: the number of adsorbed lymphocytes per unit weight of the adsorbent (number/g). When these numbers of the adsorbed blood cells are measured, a quantitative determination of the blood cells and a hematocrit value can be measured by using XT-1800iV supplied from Sysmex. Here, the number of the granulocytes is calculated in terms of a neutrophil count. When the cell adsorption column of the present invention is used for the purpose of evaluating by the above formulae (1) and (2), a blood flow rate is 30 mL/minute.

One example of the evaluation by the above formulae (1) and (2) is as follows. When an experiment is performed by directly collecting blood from a subject (e.g., a human healthy donor), the blood (each 1 mL) is collected at four points: 5, 15, 30 and 60 minutes after the circulation is started, before and after the blood passes through the column. And then, the numbers of the blood cells are counted in each sample. Dt, which indicates the number of adsorbed cells per unit weight of the adsorbent in 1 mL of the blood at a time (referred as t minutes) of the sampling point, is calculated by the following formula.

```
Dt (number/g) = [(Blood cell count in blood 1 mL
  before circulation in column) - (Blood cell count in blood
  1 mL after circulation in column)]
```

The number D of the adsorbed blood cells per unit weight of the adsorbent is calculated by the following formula.

```
D (number/g) =
30×(5×D5+10×D15+15×D30+30×D60)/Weight of adsorbent (g)
```

When the numbers of adsorbed blood cells are measured, an experiment which applies a size of the cell adsorption column of the present invention (actual size) by using the human blood might be ethically difficult. Thus, it is desirable in some cases to perform the experiment by downsizing based on a miniature column method. In that case, it is proper that a blood flow rate is changed so that a contact time of the blood with the adsorbent for a unit time period relative to the absorbent-filled volume of the miniature column is equivalent to a contact time relative to the volume of the cell adsorption column (actual column) of the present invention. One example of the cases performing the evaluation by the above formulae (1) and (2) by using a miniature column is as follows. The actual column (radial flow type column having an internal diameter of 36 mm and a adsorbent-filled volume of about 70 mL) is downsized, and then the numbers of adsorbed blood cells are obtained. An adsorbent is cut into a disc shape having a diameter of 1 cm, and the pieces are laminated inside a cylindrical column (internal diameter of 1 cm) having a filled volume of 2 mL; in this way, the column is filled with the pieces. A downsized measurement is evaluated by using this column and passing 25 mL of blood collected with heparin (heparin concentration: 10 U/mL) from a human healthy donor. A blood flow rate is selected so that a contact time of the blood with the adsorbent for a unit time period relative to the adsorbent-filled volume of this cylindrical column is equivalent to the contact time in the actual column; as the result, the blood flow rate becomes 0.86 mL/minutes.

The adsorbent is cut into the disc shape having the diameter of 1cm, and the pieces are laminated inside the cylindrical column (internal diameter of 1 cm) having a filled volume of 2 mL; then, the column is filled with the pieces. Using this column, 25 mL of human blood collected with heparin (heparin concentration: 10 U/mL) is circulated at 0.86 mL/minutes at 37°C for one hour. After that, blood samples of 1 mL are collected at each time, at the starting time for the circulation (0 minute) and at the end of the circulation (one hour). Then, the numbers of the blood cells are measured based on a blood composition. The number of adsorbed blood cells per unit weight of the adsorbent in 1 mL of the blood at a time (referred as t minute) of the sample collection is Dt, and the number D' of the adsorbed blood cells per unit weight of the adsorbent is obtained by the following formula.

```
D' (number/g)=25×(D0-D60)/Weight of adsorbent (g)
```

In the present invention, it is possible to obtain a, b and c by the above two methods. The formula (1) can be evaluated in turn as that a/2c exceeds 1 (a/2c>1), and a/2c is obtained by rounding off to one decimal place. Also, it is sufficient that b satisfies the formula (2) in any one of the above scale experiments.

A reason why it is desirable that the number b of adsorbed monocytes in the formula (2) exceeds $10^6$ in the cell adsorption column of the present invention is as follows. That is, it has been found that increased monocytes are closely associated with the activation of immune cells especially in inflammatory diseases; therefore, removal of these monocytes from blood is preferable. When only monocytes less than $10^6$ are removed per a unit weight of the adsorbent (g), the remaining monocytes are too many, as a result, an sufficient action to suppress inflammation becomes reduced. Thus, this is not preferable.

Furthermore, it is desirable that the number of the adsorbed lymphocytes is lower than the number of the adsorbed granulocytes. This is because the lymphocyte has important roles in antibody production, information memory and signal transduction. A ratio of a granulocyte count to a lymphocyte count in healthy human donors is individually different; that is, the ratio is observed from an equivalent to a several times higher ratio of the granulocytes. However, it is preferable that the number of adsorbed granulocytes and the number of adsorbed lymphocytes per unit weight of the adsorbent satisfy a relationship of the above formula (1) when the cell adsorption column of the present invention is used, and it is more preferable to satisfy 4c<a in place of the formula (1). The lymphocyte referred to here is a generic name of the so-called lymphocytes. In other words, it is referred to as the lymphocyte in accordance with the cell classification using XT-1800iV supplied from Sysmex, but it does not intend to distinguish B cells and T cells.

In the cell adsorption column, if a volume to fill with an adsorbent is increased, operability worsens because an amount of blood treated at a time is increased, and it also causes concerns such as denaturation and differentiation induction in blood due to a contact of the blood with the adsorbent because a residence time of the blood in the column is increased. The cell adsorption column of the present invention is **characterized in that**: the volume to fill the column with the adsorbent is 100 mL or less; the adsorbent filling the column contains fibers having a fiber diameter of 0.5 $\mu$m or more and 10 $\mu$m or less; and a surface area of the adsorbent is 0.5 $m^2$ or more and less than 10 $m^2$. The column having this characteristic can solves the aforementioned problems.

It is preferable that a lower limit of the adsorbent-filled volume is 2 mL or more depending on an filling amount of the adsorbent, preferably 5 mL or more in terms of preventing clogging when blood passes through the column, and more preferably 10 mL or more for safety.

A size of the cell adsorption column of the present invention is not particularly limited. However, it is inefficient in some cases even though the cell adsorption column is compact, if the number of the columns to be used is increased in order to treat a unit amount of blood. That is, when the cell adsorption column of the present invention is utilized commercially and industrially, it is advantageous that a blood amount which can pass through the column is more than a certain amount. Specifically, when the total amount of blood treated for cell removal without causing harmful effects such as hemolysis due to an increase of column pressure under substantial pressure loss is represented as a blood treatment amount, it is desirable that the blood treatment amount of the cell adsorption column of the present invention is 1500 mL or more. This is more preferably 1800mL or more for making sufficient, and still more preferably 2000mL or more for the safety. A blood treatment amount can be determined by the pressure loss between the front and the rear of the column (inlet and outlet of the column) when the predetermined amount of blood passes through the column. For example, in a case of an extracorporeal circulation experiment applying to a healthy dog, it is preferable that the pressure loss is 150 mmHg or less, when the hematocrit value of the blood is adjusted to 40% using saline and 1500 mL of the blood treatment amount passes through the column at a rate of 30 mL/minute. The healthy dog means a dog in a healthy state, and being healthy can be confirmed by no weight loss during a pre-breeding period for a week. As a dog, a male dog having a body weight of 9 to 12 kg may be used, as used in Examples. A kind of dogs is not particularly limited, for example, a beagle dog may be used.

As to the above mention, more details are described as follows. Since the monocyte has a strong foreign substance recognition ability, it is advantageous for the removal thereof that the surface area of the adsorbent in the column is large. Thus, it seems to be effective for the removal of monocytes at a glance that the surface area of the adsorbent is made large. However, if an filled amount of the adsorbent is simply increased, a risk to cause clogging in a column is increased during the circulation of blood. In order to prevent it, if a volume for filling the column is excessively increased, there is concern about denaturation and differentiation induction of cells caused by a contact of the cells with the adsorbent. Therefore, this is difficult to be employed. On the other hand, the fiber diameter of the fibers contained in the adsorbent is controlled to 0.5 $\mu$m or more and 10 $\mu$m or less in the cell adsorption column of the present invention. Therefore, the foreign substance recognition ability of the monocyte can be utilized effectively and also a phagocytic action of the granulocytes can be utilized efficaciously. Thus, the column produces the large difference between the number of the adsorbed monocytes and the number of the adsorbed lymphocytes. As the result, even when the column is made compact where the volume for filling the adsorbent is 100 mL or less, it is possible to realize the cell adsorption column having the required foreign substance recognition ability. A foreign substance recognition ability and phagocytosis of monocytes work more strongly on the substances of the size of 0.5 $\mu$m or more and 10 $\mu$m or less *in vivo*. Thus, as a fiber which composes the adsorbent of the cell adsorption column of the present invention, it is preferable to use the fiber having a fiber diameter in this range which mimics sizes of the foreign substances. However, when the fiber diameter is thin, dispersion of the fibers per se becomes worsened due to an elasticity shortage of the fibers per se, and it is likely that the fiber can not work advantageously as the substance to be recognized by the monocyte and the granulocyte. Thus, in order to stabilize an adsorbability, it is preferable that a fiber-diameter of the fiber in the adsorbent is 3 $\mu$m or more and 10 $\mu$m or less. This is preferable also in order to increase the difference from the number of the adsorbed lymphocytes.

Here, the fiber diameter in the present invention refers to a diameter of the fiber cross-section. The fiber diameter is obtained by randomly collecting 10 small piece samples from the adsorbent, taking photographs at magnifications of 1000 to 3000 times using a scanning electron microscope (JSM-5400LV supplied from JEOL [Japan Electron Optics Laboratories] Ltd.), measuring the diameters of total 100 fibers from each sample by 10 fibers and calculating by rounding off to the whole number.

It is preferable that the surface area is less than 10 $m^2$ when the column container is filled with the adsorbent containing the fibers having the aforementioned diameters. This comes from concerns such as denaturation and differentiation induction of cells caused by a contact of the adsorbent with the cells. Moreover, it is preferable for safety that the surface area is less than $8m^2$. It is also necessary that the surface area of the filled adsorbent is 0.5 $m^2$ or more. If the surface area is smaller than this, $10^6$ or more monocytes can not be adsorbed. In order to adsorb $10^6$ or more monocytes stably, it is preferable to be 1.0 $m^2$ or more.

For an evaluation of the surface area referred to here, using mercury porosimetry method is more the simple way to be applied. In the present invention, the pressure is measured in a range of 0.4 to 3000 psia. Additionally, it is also possible to measure using the BET method.

The adsorbent-filled volume referred to in the present invention means a volume of an entire space which is filled with the adsorbent in the cell absorbent column. In other words, it means an entire internal volume in the state where the blood flow inlet and outlet are closed, and the volume can be calculated from the amount of water whose specific gravity is known, which can be filled in the cell adsorption column (mainly in the column container) in the state where matters such as the adsorbent and the filter to be filled are removed. When the filled materials can not be removed, the volume can be obtained by measuring an internal shape of the column container in detail and calculating from the measured values.

The cell adsorption column of the present invention can be produced by filling the aforementioned adsorbent in the column container. As the column container, those obtained by adjusting a publicly known column container used for blood treatment to the volume for filling with the adsorbent can be appropriately selected and used. As a component of the column, following columns are preferable: (1) a column where adsorbents formed into a plate shape are laminated to fill, (2) a column where a cylindrical filter obtained by rolling an adsorbent up to a cylindrical shape is housed in a cylindrical container having the blood flow inlet and the blood flow outlet at both ends, and (3) a column where a hollow cylindrical filter obtained by rolling an adsorbent into a hollow cylindrical shape in a state where its both ends are sealed is housed in a cylindrical container having the blood inlet and the blood outlet, wherein the blood flow outlet of the container is provided at a part that leads to an outer periphery portion of the hollow cylindrical filter and the blood flow outlet of the container is provided at a part that leads to an inner periphery portion of the hollow cylindrical filter. Among them, the column (3) using the hollow cylindrical filter is the most preferable because a majority of cells in blood is rapidly removed by a nonwoven fabric having a large area in the outer periphery part of the cylindrical filter, remaining few cells which are not removed are removed by the nonwoven having a small area in the inner periphery part of the hollow cylindrical filter, and thus the cells can be adsorbed efficiently.

In order to efficiently adsorb and remove cells such as a granulocyte and a monocyte by the cell adsorption column of the present invention, it is preferable that a polymer which composes the adsorbent is a water-insoluble carrier. In this case, it is also preferable that the diameter of the fibers or hollow fibers contained in the adsorbent is 0.5 $\mu$m or more as described above. When the diameter becomes smaller than this, elastic force and firmness of the fibers are lost and air gaps formed by the fibers one another can not be assured stably. As the result, the number of the adsorbed and removed lymphocytes is increased, which leads to the removal of memory cells. Thus, this is not preferable. But, in order to reduce a percentage of the adsorbed and removed lymphocytes, a range of the fiber diameter is more preferably 3 $\mu$m or more and still more preferably 4 $\mu$m or more. Furthermore, it is more preferable in some cases that the fiber diameter is 5 $\mu$m or more from a viewpoint of reducing a percentage of the removed lymphocytes and not leading to a reduced percentage of the removed granulocytes and monocytes. However, when the fiber diameter exceeds 8 $\mu$m, the percentage of removed granulocytes and monocytes tends to be reduced. When the fiber diameter exceeds 10 $\mu$m, the percentage of removed granulocytes and monocytes is reduced and the performance is reduced. Thus, it is preferable from a practical viewpoint that the fiber diameter is 20 $\mu$m or less.

Preferable materials as the water-insoluble carrier are hydrophobic fibers, that is, polyolefin such as polyethylene and polypropylene, polyester such as polyethylene terephthalate and polybutylene terephthalate, and fluorinated polymers including polytetrafluoroethylene. Also, those obtained by surface modification by adding various alkyl groups and aromatic rings to provide hydrophobic sites may be used as described later. As suitable specific examples of the polymers on which an amine residue is immobilized may include polysulfone based polymers represented such as poly(p-phenylene ether sulfone) : $-\{(p-C_6H_4)-SO_2-(p-C6H4)-O-\}n-$ and UDEL polysulfone: $-\{(p-C_6H_4)-SO_2-(p-C_6H_4)-O-(p-C_6H_4)-C(CH_3)_2-(p-C_6H_4)-O\}_n-$, and additionally $-\{(p-C_6H_4)-SO_2-(p-C_6H_4)-O-(p-C_6H_4)-O\}_n-$, $-\{(p-C_6H_4)-SO_2-(p-C_6H_4)-SO_2-(p-C_6H_4)O\}_n-$ and $-\{(p-C_6H_4)-SO_2-(p-C_6H_4)-O-(p-C_6H_4)-C(CF_3)_2-(P-C_6H_4)-O\}_n-$; polyether imide; polyimide; polyamide; polyether; polyphenylene sulfide; polystyrene; and acrylic polymers, which have a reactive functional group(s) capable of covalently immobilizing the amine residue described later, are used simply and easily. Among them, polystyrene and the polysulfone based polymers are preferably used because their stability is high and their shape retention is good.

As specific examples, chloroacetamidemethylated polystyrene, chloroacetamidemethylated UDEL polysulfone and chloroacetamidemethylated polyether imide, which have been bound to the aforementioned reactive functional group are used. Among these polymers, those which are soluble in an organic solvent(s) are particularly preferably used in light of ease for molding processes including that a coating step can be employed.

As the adsorbent used in the present invention, those where the amine residue as the functional group has been coupled to the polymer (e.g., the aforementioned water-insoluble carrier) which composes the adsorbent are preferable. Among them, those where a quaternary ammonium group or a straight amine group or both groups thereof (referred to as the quaternary ammonium group and the like) are immobilized are preferable. It is preferable that that these functional groups are coupled to the water-insoluble carrier which composes the adsorbent. The reactive functional groups for immobilizing the quaternary ammonium group to the water-insoluble carrier may include active halogen groups such as halomethyl groups, haloacetyl groups, haloacetamidemethyl groups and halogenated alkyl groups; epoxide group; carboxyl group; isocyanate group; thioisocyanate group; and acid anhydrate groups. Particularly, the active halogen groups are preferable. Among them, the haloacetyl group is preferable because its production is easy, its reactivity is appropriately high, an immobilization reaction of the quaternary ammonium group and the like can be carried out under a mild condition as well as a covalent bond produced at that time is chemically stable.

The quaternary ammonium group and the like mean the quaternary ammonium group and/or primary to tertiary amino groups. As the primary to tertiary amino groups, those having 18 or less carbon atoms per nitrogen atom are preferable for enhancing a reaction rate. Furthermore, among the primary to tertiary amino groups, the adsorbent where the tertiary amino group having an alkyl group having 3 or more and 18 or less carbon atoms, particularly 4 or more and 14 or less carbon atoms per nitrogen atom is coupled (immobilized) to the polymer is excellent in terms of cytokine adsorbability. Specific examples of such a tertiary amino group may include trimethylamine, triethylamine, N,N-dimethylhexylamine, N,N-dimethyloctylamine, N,N-dimethyllaurylamine and N-methyl-N-ethyl-hexylamine. Examples of compounds having a straight amino group may include tetraethylenepentamine. These amino groups include those having a partially branched structure.

A density of binding of the quaternary ammonium group and the like to the adsorbing-material in the present invention varies depending on a chemical structure and a use of the polymer such as the water-soluble carrier and the like which composes the adsorbing-material. When the density is too low, its function tends not to be exerted. On the other hand, when it is too high, physical strength of the carrier after the immobilization is decreased and the function as the adsorbent tends to decrease. Thus, the density is 0.01 to 2.0mol and preferably 0.1 to 1.0mol per repeating unit of the water-insoluble carrier. As a method of immobilizing (quaternization) the quaternary ammonium group and the like, a reaction using potassium iodide as a catalyst is often used, but without being restricted by this, it is also possible to use publicly known methods.

In the present invention, the method of coupling the amine group of the quaternary ammonium group and the like to the water-insoluble carrier as described above is not particularly limited to the aforementioned method. For example, it is easily produced by dissolving a polymer solution containing the amine residue of the quaternary ammonium group and the like in a solvent such as methylene chloride, tetrahydrofuran or N,N-dimethylformamide, immersing a nonwoven composed of the water-insoluble carrier in this solution and subsequently evaporating the solvent.

Meanwhile, in the adsorbent of the present invention, a hydrophobic site may be imparted in conjunction with, or in place of immobilizing the functional group to the polymer such as the water-insoluble carrier which composes the adsorbent as described above. The hydrophobic site is, for example, the group containing an alkyl group (ethyl, octyl, hexyl, lauryl) or an aromatic group, and can be imparted and introduced to the polymer by an organic reaction.

The cell adsorption column of the present invention can adsorb cytokines. The cytokine is at least one cytokine selected from the group consisting of interleukin-1 (IL-1), interleukin-6 (IL-6), interleukin-8 (IL-8), interleukin-10 (IL-10), tumor necrosis factor-$\alpha$ (TNF-$\alpha$), transforming growth factor-$\beta$ (TGF-$\beta$), vascular endothelial growth factor (VEGF) and immunosuppressive acidic protein (IAP), and may be a combination of two or more thereof. These all are the cytokines indicated to be involved in pathological conditions of ulcerative colitis, Crohn's disease and chronic rheumatoid arthritis, for which the application of a leukocyte reduction therapy is considered.

In the cell adsorption column of the present invention, the amine group of the quaternary ammonium group and the like to be immobilized to the adsorbent may be appropriately selected depending on the type of the cytokine to be adsorbed among them. When the cytokine such as interleukin-1 (IL-1), interleukin-6 (IL-6), transforming growth factor-$\beta$ (TGF-$\beta$), vascular endothelial growth factor (VEGF) or immunosuppressive acidic protein (IAP) is targeted, the adsorbability can be imparted to the resulting cell adsorption column by immobilizing the amine group such as N,N-dimethylhexylamine, N,N-dimethyloctylamine or N,N-dimethyllaurylamine. When the cytokine such as interleukin-8 (IL-8), interleukin-10 (IL-10), transforming growth factor-$\beta$ (TGF-$\beta$) or tumor necrosis factor-$\alpha$ (TNF-$\alpha$) is targeted, the adsorbability can be imparted to the resulting cell adsorption column by immobilizing tetraethylenepentamine as the amine component to the adsorbent. Multiple types of the functional groups may be combined and immobilized. For example, both of the quaternary ammonium salt and the straight amine group may be used. By the use of the combination of the multiple type functional groups, it is possible to extend the range of cytokine types to be adsorbed; thus, this is preferable, and this is advantageous in that an adsorbable property of the desired cytokine is enhanced.

As described above, the shape of the adsorbent is not particularly limited. It is preferably a fiber, a membrane or a hollow fiber when used as the column. The form is preferably a fiber structure such as knits, fabrics and nonwovens obtained by knitting or texturing the fibers. If the adsorbent itself can keep its morphology, the adsorbent alone can be used. If its morphological retention is low, the adsorbent can be secured to an appropriate substrate by coating, or can be mixed with other adsorbent to used as one column. Securing and mixing may be performed before the adsorbent is processed into the above shape.

The nonwoven in the present invention may be produced from a single fiber, but is preferably produced from a sea-island type composite fiber. That is, the water-insoluble carrier can be easily produced by making a nonwoven by the publicly known method using the composite fiber, giving a needle punch to the nonwoven for enhancing the morphological retention and adjusting the bulk density, and subsequently dissolving a sea component.

The method for producing the adsorbent of the present invention will be mimetically described employing the nonwoven as an example for the form of the adsorbent. When multiple types of the fibers having different fiber diameters are used as materials, the fibers are weighed so as to achieve a target mixing-ratio, and passed through a card in a mixed state to disperse mutually and sufficiently in a flocculent state.

The nonwoven is made by weighing this flocculent product to have a target mass per unit area, subsequently passing it through a cross-lapper and giving the needle punch to it. For morphological stabilization, this nonwoven and a net made separately may be laminated to make a laminate structure by a publicly known web adhesion method such as a thermal bond method, a calendar method and a needle punch method. The more preferable method of making the laminate structure is the method: the flocculent product previously given a pre-punching is made and the net is inserted therein, and then the punching is given, to make the adsorbent having the laminate structure of nonwoven-net-nonwoven. This method is simple and easy, and thus is suitable for continuous production. It is also possible to produce the adsorbent having a multilayer structure by laminating a bilayer structure obtained by placing one net on one side of the pre-punched flocculent product. The morphological retention is improved by making a two or more layer structure with the net. The adsorbent may be the bilayer structure of the nonwoven and the net, and is more preferably the structure where the net is inserted between the nonwovens, i.e., the sandwich structure of nonwoven-net-nonwoven. Obviously it is possible to make a more multilayer structure within the range in which pressure loss between the front and the rear of the adsorbent is not affected when a medium to be treated is passed through with considering a bulk density of the adsorbent.

As a material for the net in the present invention, publicly known polymers such as polyamide, polyester, polyacrylonitrile based polymers, polyethylene and polypropylene (PP) may be used. As described later, when the net is integrated with the nonwoven and subsequently is subjected to an organic synthesis reaction for introducing the functional group, the material may be appropriately selected depending on a type of the solvent used and a reaction temperature. In particular, PP is preferable in terms of biocompatibility and steam sterilization resistance. Polyester and polyethylene are preferable when radiation sterilization is performed.

It is preferable that the net is formed of a monofilament because if the net structure is formed of a multiple wound yarn made from multiple fibers or a spun yarn, there is concern about increase of the pressure loss due to passing the medium to be treated, e.g., the blood through spaces on the yarns. If it is a monofilament, a mechanical strong force per one is also maintained easily.

A diameter of the monofilament is preferably 50 $\mu$m or more and 1 mm or less, and likewise a thickness of the net is preferably 50 $\mu$m or more and 1.2 mm or less. Such range and net in the larger range than them can be used, but this is not preferable because an amount of the adsorbent itself per unit area is decreased.

A constitution of the net is not particularly limited, and nets such as a knotting net, a non-knotting net and a raschel net may be used. A shape of the mesh (void shape) in the net is not particularly limited, and a rectangle, a rhombus and a tortoise shell shape may be used. Furthermore, for a positional relationship of the net component with the nonwoven, it is preferable to laminate the net on the nonwoven so as to make a direction of an angle 90 degrees $\pm$ 10 degrees against a major or minor axis of the nonwoven when viewed from major surfaces of the net and the nonwoven in a case where the void shape of the net is a quadrangle. This enhances the strength and handling performance when the nonwoven is laminated, and also enhances a yield (yield ratio) upon production.

A use of the net imparts a morphological retention to the nonwoven, and can make the morphologically stable adsorbent even when a bulk density is small. Since the net itself affects pressure loss of the medium to be treated, it is desirable that openings in the net are as large as possible. Thus, it is preferable to have the void of 10 $mm^2$ or more in any area of 100 $mm^2$. In particular, the net having openings of about 3 mm square exhibits a good morphological retention and can be used suitably.

It is particularly preferable that the form of the adsorbing-material is a nonwoven. In that case, when a bulk density of the nonwoven is too large, clogging easily occurs; on the other hand, when it is too small, a morphological retention becomes poor. Thus, the bulk density is preferably 0.02 to 0.15 $g/cm^3$ and more preferably 0.05 to 0.15 $g/cm^3$.

The bulk density in the fiber portion where its fiber diameter is 0.5$\mu$m or more and 10$\mu$m or less is preferably 0.01 g/cm$^3$ or more in the adsorbent, and it is more preferably 0.03 g/cm$^3$ or more. To prevent the clogging, the bulk density is preferably 0.15 g/cm$^3$ or less and more preferably 0.13 g/cm$^3$ or less. The bulk density referred to here indicates a bulk density of the adsorbent in the final stage, which is a sheet processed into a felt shape obtained after introducing the desired functional group. When a bulk density is increased, an ability to filtrate large substances such as leukocytes and cells is enhanced, but when it is too large, clogging occurs easily upon blood circulation. Thus the bulk density in the above range is preferable.

The bulk density can be measured as follows. The adsorbent is cut into a small piece of a square with 3cm square, a polypropylene (PP) plate having 5cm square and its thickness of 1 mm is placed on the adsorbent like being overlapped in a thickness direction, and a thickness of the adsorbent in this state is measured. The plate is removed, placed again and the thickness of the adsorbent is measured. This manipulation is repeated five times, and an average value thereof is evaluated as the thickness. A bulk density is obtained by dividing a weight of the small piece by a volume calculated from the above thickness. This measurement of bulk density is performed in five samples, and an average value is evaluated as the bulk density. In the case of having the net, a bulk density is likewise obtained by calculating from a volume calculated by measuring a thickness of the small piece having the net, and a weight obtained by subtracting the weight of the fiber having the fiber diameter of 0.5 $\mu$m or more and 10 $\mu$m or less and the weight of the net from the weight of small pieces. When the column container is filled with the adsorbent, it is necessary to fill so that the bulk density is not changed.

It can be accomplished by controlling the bulk density described above that no clogging occurs when an amount of the blood passed through the column is up to 1500mL or more.

One utilization example of the cell adsorption column of the present invention will be described. The column of the present invention is composed of the container having the flow inlet and the flow outlet. The container is filled with the adsorbent for cell adsorption, and it is constituted to have a flow path to lead the blood before and after the contact to the adsorbent for the cell adsorption. The flow path is communicated with both the flow inlet and flow outlet. Body fluid which flows in from the flow inlet flows through the flow path, contacts with the adsorbent for the cell adsorption before or after thereof, and flows out from the flow outlet.

The flow inlet and the flow outlet are the means to run body fluid in or out from the cell adsorption column used for extracorporeal circulation, and are the sites to be connected to a circuit of a circulation system when used for the extracorporeal circulation therapy. A shape is not particularly limited, but it is desirable that the shape is a form capable of being connected to a tube which forms the circuit for the extracorporeal circulation. More preferably, it is desirable that the shape has a connector shape and is easily removable from the tube of the circuit.

The above flow path has an action to lead blood to the inside of the column or lead blood supplied to the column out of the column, and the leading action allows blood to be uniformly supplied to and contacted with the adsorbent for cell adsorption. If a column does not have this flow path for leading, a drift flow occurs in some cases depending on an conformation of filling the adsorbent for cell adsorption. When the drift flow occurs, body fluid can not be contacted uniformly with the filler for cleanup and this causes a reduction of the cleanup ability.

A shape of the flow path is not particularly limited as long as it has the leading action of body fluid. For example, pipes having a cross-sectional shape of a circle, a square, a triangle and other polygons and the combinations thereof, a shaped body obtained by folding a thin bar into a cube type and stacking it, a bundling body obtained by bundling hollow fibers, and a space formed by once covering over a periphery of a pipe to form the flow path and subsequently removing the pipe, can be included. As a shape in a longitudinal direction of the flow path, a straightforward shape in straight line is preferable not to cause a retention of body fluid. Also the shape having a moderate taper along a flow direction is preferable.

One example of a constitution of the inside of the cell adsorption column of the present invention will be described along FIG. 1. In FIG. 1, 1 represents the container main body, the front end and the back end in its longitudinal direction are provided with the flow inlet 2 and the flow outlet 3. The filter 4 and the disc-shaped divider plate 5 are provided inside the flow inlet 2. The filter 6 and the disc-shaped divider plate 7 are provided inside the flow outlet 3.

In the two divider plates 5 and 7, the center portion of the divider plate 5 at the front side is provided with the opening 5a, and the center portion of the divider plate 7 at the back side is provided with the support lug 7a. Many through holes 7b are intermittently provided at the outer periphery of the divider plate 7 in line with its circumferential direction. Furthermore, one pipe 8 is bridged between the opening 5a in the divider plate 5 and the support lug 7a in the divider plate 7.

The above pipe 8 forms the flow path 9 for leading blood inside thereof and provides the many penetration holes 10 on its peripheral wall. The front end of the pipe 8 is communicated with the opening 5a in the divider plate 5 as well as the back end of the pipe 8 is closed by the support lug 7a in the divider plate 7. The adsorbent 11 in multilayer of numerous layers is wrapped around the outer periphery of this pipe 8.

When this cell adsorption column is used for a circulation method, the tubes which have formed the circulation circuit with the blood pool are connected to the flow inlet 2 and the flow outlet 3, and blood is circulated so that the blood run from the blood pool is supplied to the flow inlet 2, harmful substances are removed by the inside adsorbent 11 and the blood is run out from the flow outlet 3 to return the blood pool.

In the column, blood infiltrates in the flow path 9 through the filter 4 from the flow inlet 2 sequentially infiltrates in the adsorbent 11 through the penetration holes 10 with moving in the flow path 9, and cells and the like are adsorbed as blood moves to any of radius directions. The blood from which the cells and the like have been removed flows out from the many through holes 7b in the outer periphery of the divider plate 7, and flows out from the flow outlet 3 through the filter 6.

In the above example, as the blood flows in the flow path 9 in the pipe 8 from the opening 5a, the blood flows out from the penetration holes 10. However, by reversing the moving direction of blood in the above in the cell adsorption column of the present invention, the blood may be supplied from the flow outlet 3 and run out from the flow inlet 2.

EXAMPLES

The present invention will be described more specifically by the following Examples, but the present invention is not limited thereto.

Evaluation parameters, and conditions for the measurement and the calculation in the following Examples will be explained in advance.

(Measurement of blood cell counts)

The blood cell counts in blood were quantified and hematocrit values were measured, using XT-1800iV supplied from Sysmex. Here a granulocyte count was calculated as a neutrophil count.

(Evaluation of cytokine adsorption)

For evaluation of cytokine adsorption, human natural type IL-1 and IL-6 were added to bovine fetal serum, and prepared at 500 pg/mL, respectively. An adsorbent was added to this serum, which was then shaken at 37°C for 2hours, and a supernatant was collected to use as the sample. A ratio of solid to liquid of the adsorbent : the serum was fixed to 30 mg : 1 mL, and the amounts of the cytokine before and after the shacking were measured to obtain a cytokine adsorption rate (removal rate) by the following formula. The cytokines were quantified by an EIA method using commercially available kits (IL-1: human IL-1β ELISA kit supplied from R & D System, IL-6: kit supplied from Kamakura Techno-Science Inc.).

Cytokine adsorption rate (%) =[(Cytokine concentration in serum before shaking) - (Cytokine concentration in serum after shaking)]/(Cytokine concentration in serum before shaking) x 100

(Measurement of fiber diameter)

The fiber diameter is obtained by randomly collecting 10 small piece samples from the nonwoven, taking photographs at magnifications of 1000 to 3000 times using the scanning electron microscope (JSM-5400 LV supplied from JEOL [Japan Electron Optics Laboratories] Ltd.), measuring the diameters of total 100 fibers from each sample by 10 fibers on the basis of the photographs, and calculating by rounding off their average value to the whole number.

(Measurement of surface area)

The surface area of the adsorbent filled in a column container was evaluated using a mercury porosimetry method. The measurement was performed in the pressure range of 0.4 to 3000 psia. The dry adsorbent was weighed, and a poresizer 9320 supplied from Micrometrics was used.

(Measurement of bulk density)

After the adsorbent made in Production Example is cut into a small piece of a square with 3 cm square, a PP plate having 5cm square-form and 1 mm thickness is placed on the adsorbent like being overlapped in a thickness direction, and then the thickness of the adsorbent in this state is measured. The plate was removed and placed again, and the thickness of the adsorbent was measured. This manipulation is repeated five times, and an average value thereof was evaluated as the thickness. A bulk density was obtained by dividing the weight of this small piece by the volume calculated from the above thickness. This measurement of bulk density was performed in five samples by each adsorbent, and the average value was evaluated as the bulk density. In the case of having the net, the bulk density is likewise obtained by calculating from the volume calculated by measuring the thickness of the small piece having the net, and the weight obtained by subtracting the weight of the net from the weight of the small piece.

(Measurement of volume for filling adsorbent)

The adsorbent-filled volume (abbreviated as a "filled volume" in Table) refers to the volume of an entire space in which the adsorbent was filled. In other words, it means an entire internal volume in the state where the blood flow inlet and blood flow outlet were closed, and was calculated from the amount of water whose gravity is known, wherein the internal volume was in the state that materials such as the adsorbent and the filter to be filled were removed.

(Measurement of number of adsorbed blood cells per unit weight of adsorbent-the circulation method)

An extracorporeal circulation test was performed to healthy a dog having a body weight of about 10 kg (9 to 12 kg) (dog type: beagle, gender: male, age: half a year to 2 years old, the dogs were bred for one week after purchasing them and no weight loss was confirmed.) by using the cell adsorption column and infusing saline for adjusting the hematocrit value of the blood to 40% at the blood flow rate of 30 mL/minute. The blood (each 1mL) was collected at four points of 5, 15, 30 and 60 minutes after starting the circulation at the front and the rear of the column. The blood cell counts were measured by the method described in "Measurement of blood cell counts". The number $D_t$ which is the adsorbed cells per unit weight of the adsorbent in 1 mL of the blood at the time (t minutes) on sample collection, was obtained by the following formula.

$D_t$ (number/g) = [Blood cell count in blood 1mL before circulation in column] - [Blood cell count in blood 1mL after circulation in column]

The number D of the adsorbed blood cells per unit weight of the adsorbent is calculated by the following formula.

$D$ (number/g) = $30 \times (5 \times D5 + 10 \times D15 + 15 \times D30 + 30 \times D60)$/Weight of adsorbent (g)

In Examples described later, the pressure loss between the front and the rear of the column was measured at time points of the blood treatment amounts of 1500 mL and 1750 mL.

(Measurement of number of adsorbed blood cells per unit weight of adsorbent-the miniature column method)

This was performed by downsizing based on the miniature column method. According to a column size, the blood flow rate was set to 0.65 mL/minute corresponding to 30 mL/minute upon use of the actual column (corresponding to 100 mL of the adsorbent-filled volume).

The adsorbent in a predetermined amount was cut into a disc shape having the diameter of 1 cm, and the resulting pieces were laminated to fill the cylindrical column (internal diameter of 1 cm) having an internal volume of 2 mL. Using this column, 25 mL of the blood collected with heparin (heparin concentration: 10 U/mL) from a human healthy volunteer was circulated at the blood flow rate of 0.65 mL/minute at 37°C for one hour. A blood sample of each 1 mL was collected at the start of the circulation (0 minute) and at the end of the circulation (one hour), and the composition of the blood was measured by the method in "Measurement of blood cell counts". The number of the adsorbed blood cells per unit weight of the adsorbent in 1 mL of the blood at the time (t minute) of the sample collection was $D_t$, the number D' of the adsorbed blood cells per unit weight of the adsorbent was obtained by the following formula.

$D'$ (number/g)=$25 \times (D0-D60)$/weight of Adsorbent (g)

This test method was regarded as a downsized experiment of the circulation test, and the surface area of the adsorbent when the filling volume was 100 mL was addressed as 50 folds of the surface area of the adsorbent used in the present experiment.

In any of the above two methods, the values of a, b and c in the above formulae (1) and (2) were obtained. In the formula (1), the value of a/2c was obtained by rounding off to one decimal place.

[Production Example 1: Preparation of adsorbents 1 to 4]

(Nonwovens 1 and 2)

A sea island composite fiber having 36 islands where the island was further composed of the core/sheath composite was obtained using the following components under the condition of a spinning rate of 850 m/minute and a drawing scale of 3 times.

Core component of island; polypropylene
Sheath component of island; 90% by weight of polystyrene and 10% by weight of polypropylene
Sea component; copolymer polyester (PETIFA) which is ethylene terephthalate as the major repeating unit containing 3% by weight of 5-sodium sulfoisophthalate as a copolymerization component
Composite ratio (weight ratio); core : sheath : sea =41:41:18

A nonwoven was obtained by making a sheet-like material composed of 30% by weight of the fiber (diameter: 30 $\mu$m) and 70% by weight of polypropylene having a diameter of 22 $\mu$m, and giving the needle punch thereto (nonwoven 1). Likewise, a nonwoven was obtained by making a sheet-like material composed of 68% by weight of the fiber (diameter: 30 $\mu$m) and 32% by weight of the polypropylene (diameter: 22 $\mu$m) and giving the needle punch thereto (nonwoven 2).

(Nonwovens 1 and 2 with removal of sea component)

Subsequently, these nonwovens 1 and 2 were treated with an aqueous solution of sodium hydroxide (3% by weight) at 90°C to dissolve PETIFA of the sea component in the aqueous solution. Thus, the nonwoven 1 with removal of the sea component, of which the diameter of the core sheath fiber was 4 $\mu$m and the bulk density was 0.05 g/cm$^3$ (total bulk density 250 g/m$^2$), was made from the nonwoven 1, and the nonwoven 2 with removal of the sea component of which the diameter of the core sheath fiber was 4 $\mu$m and the bulk density was 0.05 g/cm$^3$ (total bulk density 250 g/m$^2$), was made from the nonwoven 2.

(Intermediates 1 and 2)

Subsequently, 6 g of paraformaldehyde was dissolved in a mixed liquid of 1200 mL of nitrobenzene and 780 mL of sulfuric acid at 20°C, and then the resulting mixture was cooled to 0°C, 151.8 g of N-methylol-$\alpha$-chloroacetamide was added and dissolved at 5°C or below. In this mixture, 20 g of the above nonwoven 1 and 2 with removal of the sea component were immersed, which was then left stand at room temperature for 2 hours. Subsequently, the fiber was taken out, and placed in highly excessive cold methanol to wash. The fiber after being washed was sufficiently washed with methanol, then washed with water and dried. Thus, 24 g of $\alpha$-chloroacetamidemethylated polystyrene fiber (intermediate 1) was made from the nonwoven 1 with removal of the sea component, and 26.8 g $\alpha$-chloroacetamidemethylated polystyrene fiber (intermediate 2) was made from the nonwoven 2 with removal of the sea component.

(Adsorbents 1 and 2)

The intermediate 1 and 2 at the amount of 10 g were respectively immersed in a solution in which 100 g of N, N-dimethyloctylamine and 16 g of potassium iodide had been dissolved in 720 mL of N,N-dimethylformamide (hereinafter abbreviated as DMF), and heated in a bath at 85°C for 3 hours. The resulting fiber after being heated was washed with methanol, and immersed in brine at a concentration of 1 mol/L, subsequently washed with water, and then dried under vacuum. Thus, 13.4 g of dimethyloctyl ammoniumized fiber (adsorbent 1) was obtained from the intermediate 1, and 15.8 g of dimethyloctyl ammoniumized fiber (adsorbent 2) was obtained from the intermediate 2.

(Adsorbents 3 and 4)

The intermediate 1 and 2 at the amount of 10 g were respectively immersed in a solution in which 6.3 g of tetraethylenepentamine and 7.2 g of n-butylamine had been dissolved in 500 mL of DMF, reacted at 30°C for 3 hours, then washed with DMF, subsequently washed with water and dried under vacuum. Thus, 13.9 g of tetraethylene pentaminized fiber (adsorbent 3) was obtained from the intermediate 1, and 16.6 g of tetraethylene pentaminized fiber (adsorbent 4) was obtained from the intermediate 2.

[Production Example 2: Preparation of adsorbents 5 and 6]

(Nonwovens 3 and 4)

A sea island composite fiver having 36 islands where the island was further composed of the core/sheath composite was obtained using the following components under the condition of the spinning rate of 850 m/minute and the drawing scale of 2 times.
Core component of island; polypropylene
Sheath component of island; 90% by weight of polystyrene and 10% by weight of polypropylene
Sea component; PETIFA
Composite ratio (weight ratio); core : sheath : sea =41:41:18
A nonwoven was obtained by making a sheet-like material composed of 30% by weight of the fiber (diameter: 40 $\mu$m) and 70% by weight of polypropylene (diameter: 22 $\mu$m) and giving the needle punch thereto (nonwoven 3). Likewise, a nonwoven was obtained by making a sheet-like material composed of 68% by weight of the fiber (diameter: 40 $\mu$m) and 32% by weight of polypropylene (diameter :22 $\mu$m) and giving the needle punch thereto (nonwoven 4).

(Nonwovens 3 and 4 with removal of sea component)

Subsequently, these nonwovens 3 and 4 were treated with the aqueous solution of sodium hydroxide (3% by weight) at 90°C to dissolve PETIFA of the sea component in the aqueous solution. Thus, the nonwoven 3 with removal of the sea component, of which the diameter of the core sheath fiber was 8 $\mu$m and the bulk density was 0.13 g/cm$^3$ (total bulk density 250 g/m$^2$), was made from the nonwoven 3, and the nonwoven 4 with removal of the sea component, of which the diameter of the core sheath fiber was 8 $\mu$m and the bulk density was 0.13 g/cm$^3$ (total bulk density 250 g/m$^2$), was made from the nonwoven 4.

(Intermediates 3 and 4)

Subsequently, 6 g of paraformaldehyde was dissolved in the mixed liquid of 1200 mL of nitrobenzene and 780 mL of sulfuric acid at 20°C, then the resulting mixture was cooled to 0°C, 151.8 g of N-methylol-$\alpha$-chloroacetamide was added and dissolved at 5°C or below. In this mixture, 20 g of the above nonwoven 3 and 4 with removal of the sea component were immersed, which were then left stand at room temperature for 2 hours. Subsequently, the fiber was taken out, and placed in highly excessive cold methanol to wash. The fiber after being washed was sufficiently washed with methanol, then washed with water and dried. Thus, 23.7 g of $\alpha$-chloroacetamidemethylated polystyrene fiber (intermediate 3) was made from the nonwoven 3 with removal of the sea component, and 27.1 g $\alpha$-chloroacetamidemethylated polystyrene fiber (intermediate 4) was made from the nonwoven 4 with removal of the sea component.

(Adsorbents 5 and 6)

The intermediate 3 and 4 at the amount of 10 g were respectively immersed in a solution in which 100 g of N, N-dimethylhexylamine and 16 g of potassium iodide had been dissolved in 720 mL of DMF, and heated in the bath at 85°C for 3 hours. The resulting fiber after being heated was washed with methanol, then immersed in brine at the concentration of 1 mol/L, subsequently washed with water and dried under vacuum. Thus, 12.8 g of dimethylhexyl ammoniumized fiber (adsorbent 5) was obtained from the intermediate 3, and 14.3 g of dimethylhexyl ammoniumized fiber (adsorbent 6) was obtained from the intermediate 4.

[Production Example 3: Preparation of adsorbents 7 and 8]

(Nonwovens 5 and 6)

A sea island composite fiber having 36 islands where the island was further composed of the core/sheath composite was obtained using the following components under the condition of the spinning rate of 880 m/minute and the drawing scale of 3 times.
Core component of island; polypropylene
Sheath component of island; 90% by weight of polystyrene and 10% by weight of polypropylene
Sea component; PETIFA
Composite ratio (weight ratio); core : sheath : sea =41:41:18

The sea island composite fiber at 30% by weight (diameter: 30 $\mu$m) and 70% by weight of polypropylene (diameter: 22 $\mu$m) were thoroughly mixed and dispersed using a tuft blender, and passed through a card to make the sheet-like material. Subsequently, a net (thickness: 0.4 mm, diameter of monofilament: 0.3 mm, bulk density: 75 g/m$^2$) having openings of 2 mm square and made from polyester was inserted between the sheet-like materials so that a fiber direction in the net made an angle of 5 degrees against both end axes of the sheet-like material; and the resulting material was passed through a cross-lapper and weighed to make the target bulk density, and the needle punch was given thereto. Thus, a nonwoven composed of a trilayer structure was made (nonwoven 5). Likewise, a nonwoven composed of the trilayer structure (nonwoven 6) was made from 68% by weight of the sea island composite fiber (diameter: 30 $\mu$m) and 32% by weight of polypropylene (diameter: 22 $\mu$m).

(Nonwovens 5 and 6 with removal of sea component)

Subsequently, these nonwovens 5 and 6 were respectively treated with the aqueous solution of sodium hydroxide (3% by weight) at 90°C to dissolve PETIFA of the sea component in the aqueous solution. Thus, the nonwoven 1 with removal of the sea, of which the diameter of the core sheath fiber was 4 $\mu$m and the bulk density was 0.01 g/cm$^3$ (total bulk density 80 g/m$^2$), was made from the nonwoven 1, and the nonwoven 2 with removal of the sea component, of which the diameter of the core sheath fiber was 4 $\mu$m and the bulk density was 0.05 g/cm$^3$ (total bulk density 80 g/m$^2$), was made from the nonwoven 2.

(Intermediates 5 and 6)

Subsequently, 6 g of paraformaldehyde was dissolved in the mixed liquid of 1200 mL of nitrobenzene and 780 mL of sulfuric acid at 20°C, then the resulting mixture was cooled to 0°C, 151.8 g of N-methylol-$\alpha$-chloroacetamide was added and dissolved at 5°C or below. In this mixture, 20 g of the above nonwoven 5 and 6 with removal of the sea component were immersed, which were then left stand at room temperature for 2 hours. Subsequently, the fiber was taken out, and placed in highly excessive cold methanol to wash. The fiber after being washed was sufficiently washed with methanol, then washed with water and dried. Thus, 24 g of $\alpha$-chloroacetamidemethylated polystyrene fiber (intermediate 5) was made from the nonwoven 5 with removal of the sea component, and 26.8 g $\alpha$-chloroacetamidemethylated polystyrene fiber (intermediate 6) was made from the nonwoven 6 with removal of the sea component.

(Adsorbents 7 and 8)

Ten gram of the intermediate 5 was immersed in the solution in which 100 g of N,N-dimethyloctylamine and 16 g of potassium iodide had been dissolved in 720 mL of DMF, and then 10 g of the intermediate 6 was immersed in the solution in which 100 g of N,N-dimethylhexylamine and 16 g of potassium iodide had been dissolved in 720 mL of DMF, and heated in the bath at 85°C for 3 hours. The fiber after being heated was washed with methanol, then immersed in brine at the concentration of 1 mol/L, subsequently washed with water and dried under vacuum. Thus, 13.4 g of dimethyloctyl ammoniumized fiber (adsorbent 7) was obtained from the intermediate 5, and 15.8 g of dimethylhexylamine ammoniumized fiber (adsorbent 8) was obtained from the intermediate 6.

[Production Example 4: Preparation of adsorbents 9 and 10]

Nonwovens 7 and 8, nonwovens 7 and 8 with removal of the sea component, intermediates 7 and 8, and adsorbents 9 and 10 were made in the same way as in Production Example 2 except that the amount of the fiber was increased so that the total bulk density was 330 g/m$^2$ and the punching frequency was doubled.

[Production Example 5: Preparation of columns 1 to 12]

The adsorbents 1 to 10 obtained above were cut into a dimension having a width of 47 mm and a length of 377 mm, and subsequently the adsorbents were winded about 6 times around the core material made of polypropylene having a cylindrical shape of which external diameter was 8 mm and having the holes of which diameter was 4 mm with the opening rate of 40%, and then it was introduced into a column case having the same shape as in FIG. 1 to make columns 1 to 10 (corresponding to the adsorbents 1 to 10, respectively) having an internal diameter of 36 mm and a filled volume of 70 mL.

The adsorbents 1 and 2 obtained above were cut into a dimension having a width of 47 mm and a length of 528 mm, subsequently winded about 6 times around the core material made of polypropylene having a cylindrical shape of which external diameter was 8 mm, and having holes of which diameter was 4 mm with the opening rate of 40%, and then it was introduced into a column case having the same shape as in FIG. 1 to make columns 11 and 12 having a filled volume of 95 mL.

[Examples 1 to 10]

Extracorporeal circulation tests were performed using the resulting 10 columns: the columns 1 to 8 ,11 and 12 and using dogs (body weight: 9 to 12 kg, dog kind: beagle, gender: male, age: half a year to 2 years old, the dogs were bred for one week after purchasing them and no weight loss was confirmed.). Blood flows were standardized by a direction from an outside to an inside of the column. The animal was anesthetized by administering about 25 mg/kg of sodium pentobarbital (Nembutal supplied from Dainippon Pharmaceutical Co., Ltd.) intraperitoneally. The animal was confirmed to be stilled, and then fixed on a surgical table by its dorsal side.

The blood circuit for a continuous filtration (U-525MCB: Ube Medical Inc.) was attached to the continuous blood filtration dialysis apparatus (TR-520: Toray Medical Co., Ltd.). An arterial side of the circuit including a line for injecting heparin was filled with saline (Otsuka Normal Saline: Otsuka Pharmaceutical Factory Inc.) not to leave air bubbles, and then connected to an entrance side of the column not to bring the air bubbles, and further a venous side of the circuit was connected to an exit side of the column. The entrance side of the column was directed downward, and 2 L of saline was run using a blood pump (flow rate: 100 mL/minute). Subsequently, the column was turned upside down so that the entrance side of the column was set upward, and heparin-added saline prepared by adding 5000 U heparin (sodium heparin for injection, "Shimizu": Shimizu Seiyaku Co., Ltd.) to 500 mL of saline was run (flow rate: 50 mL/minute). A 50 mL volume syringe in which about 30 mL of heparin-added saline at 200 U/mL had been placed was set in the line for injecting heparin in the arterial side circuit.

Before a procedure, the hematocrit value was measured using the blood collected from an antebrachial vein (i-STAT: Fuso Pharmaceutical Industries Ltd.), and an infusion amount was calculated, and then saline was infused from a cephalic vein so that the hematocrit value before starting the circulation was 40%. As the infusion during the circulation, 40 mL of saline was infused from the cephalic vein over one hour.

After confirming that the anesthesia in the dog was stable, a left femoral region of the dog, which had a body weight around 10 kg, was cut out to expose femoral artery and vein, which were then connected to the blood circuit for the continuous filtration using cannula (Surflow dwelling needle 14Gx2" or 16Gx2": Terumo Corporation).

A blood circulation pump and a heparin-added syringe pump were started to initiate the blood circulation. In the blood circulation, an initial value was set at 20 mL/minute, after 5 minutes the flow was set at 30 mL/minute, and the circulation was performed for 60 minutes. From the front site of an anticoagulant injection line just before the column entrance in the blood circulation, 1500 U/body of heparin was administered once, and subsequently 140 U/kg/hr of heparin was continuously administered. At the initiation of the blood circulation and at the end thereof (one hour after the initiation), 1 mL of blood was collected from the blood collection buttons present in the blood circuit just in front of and behind the column to use as a blood sample. After completion of the above manipulation, the cannula was removed from the blood vessels, and then these blood vessels were ligated. Sodium ampicillin (viccillin for injection: Meiji Seika Kaisha Ltd.)-saline solution (one vial of viccillin for injection, titer: 500 mg) was sprayed and gentamicin sulfate (gentacin ointment 1 mg titer: Schering-Plough K.K) was applied to the cut out portion, and the skin was stitched together surgically. During the experiment, the dogs were warmed using the warmer (Natsume Seisakusho Co., Ltd.) for preventing the reduction of body temperature. After confirming that the dogs awoke from the anesthesia, they were put back to the original cages. According to "Measurement of number of adsorbed blood cells per unit weight of adsorbent, the circulation method" described above, the numbers of adsorbed blood cells were calculated using the collected blood samples, and the results were shown in Table 1. Also in Table 1, the surface area, the filled volume, the fiber diameter, the adsorbing functional group, the adsorbent weight, the adsorbent number, the pressure loss and the bulk density of the adsorbent in the column used in each Example are shown together.

[Comparative Example 1 and 2]

The extracorporeal circulation tests using the dog were performed on the resulting two columns 9 and 10, as in the same way in Examples 1 to 10. The results were shown in Table 1. Also in Table 1, the surface area, the filled volume, the fiber diameter, the adsorbing functional group, the adsorbent weight, the adsorbent number, the pressure loss and the bulk density of the adsorbent in the column used in each Comparative Example are shown together.

Table 1

| Examples/ Comp. Ex. | | Surface Area (m²) | Filled Volume (ml) | Adsorbed Blood Cells Numbers (Numbers/Adsorbent Weight (g)) | | | | Fiber Diameter ($\mu$ m) | Adsorbing Functional Group | Adsorbent Weight (g) | Adsorbent Number | Pressure Drop (mmHg) | | Adsorbent Bulk Density (g/cm³) |
|---|---|---|---|---|---|---|---|---|---|---|---|---|---|---|
| | | | | Granu-locytes (a) | Mono-cytes (b) | Lympho-cytes (c) | a/2c (> 1) | | | | | 1500ml | 1750ml | |
| Examples | 1 | 0.6 | 70 | $5.2 \times 10^7$ | $4.5 \times 10^6$ | $8.2 \times 10^6$ | 3.2 | 4.2 | Dimethyloctylamine Group | 4.8 | 1 | 42 | 53 | 0.02 |
| | 2 | 1.8 | 70 | $2.1 \times 10^8$ | $1.9 \times 10^7$ | $1.9 \times 10^7$ | 5.5 | 4.2 | Dimethyloctylamine Group | 5.9 | 2 | 67 | 88 | 0.05 |
| | 3 | 0.7 | 70 | $2.3 \times 10^7$ | $1.9 \times 10^6$ | $7.6 \times 10^6$ | 1.5 | 4.3 | Tetraethylenpentamin Group | 5.2 | 3 | 51 | 55 | 0.02 |
| | 4 | 2.2 | 70 | $8.2 \times 10^7$ | $7.6 \times 10^6$ | $1.8 \times 10^7$ | 2.3 | 4.3 | Tetraethylenpentamin Group | 6.1 | 4 | 77 | 86 | 0.05 |
| | 5 | 1.8 | 70 | $2.0 \times 10^7$ | $2.1 \times 10^6$ | $2.6 \times 10^6$ | 3.8 | 8.5 | Dimethylhexylamine Group | 4.9 | 5 | 56 | 71 | 0.09 |
| | 6 | 4.8 | 70 | $3.4 \times 10^7$ | $2.9 \times 10^6$ | $3.3 \times 10^6$ | 5.2 | 8.4 | Dimethylhexylamine Group | 5.8 | 6 | 61 | 77 | 0.15 |
| | 7 | 0.4 | 70 | $4.3 \times 10^7$ | $3.1 \times 10^6$ | $7.8 \times 10^6$ | 2.8 | 4.2 | Dimethyloctylamine Group | 4.1 | 7 | 40 | 60 | 0.01 |
| | 8 | 1.2 | 70 | $5.1 \times 10^7$ | $3.9 \times 10^6$ | $7.9 \times 10^6$ | 3.2 | 4.2 | Dimethylhexylamine Group | 4.3 | 8 | 38 | 49 | 0.01 |
| | 9 | 0.9 | 95 | $6.7 \times 10^7$ | $6.2 \times 10^6$ | $9.1 \times 10^6$ | 3.7 | 4.2 | Dimethyloctylamine Group | 7.2 | 1 | 89 | 108 | 0.03 |
| | 10 | 2.7 | 95 | $2.8 \times 10^7$ | $2.5 \times 10^7$ | $3.8 \times 10^7$ | 3.7 | 4.2 | Dimethyloctylamine Group | 8.9 | 2 | 92 | 102 | 0.05 |
| Compar-ative Exam-ples | 1 | 3.3 | 70 | $2.4 \times 10^7$ | $9.2 \times 10^6$ | $1.6 \times 10^7$ | 0.75 | 8.5 | Dimethylhexylamine Group | 9.6 | 9 | 122 | 156 | 0.18 |
| | 2 | 9.2 | 70 | $2.6 \times 10^7$ | $1.9 \times 10^7$ | $2.2 \times 10^7$ | 0.60 | 8.4 | Dimethylhexylamine Group | 11.2 | 10 | 151 | 240 | 0.30 |

※"Comp. Ex."means Comparative Examples

[Examples 11 to 18]

From a healthy volunteer, 50 mL of the blood was collected with heparin (heparin concentration: 10 U/mL), in which human natural type IL-1 and IL-6 were dissolved at 500 pg/mL, and the adsorption of cytokines was evaluated by using the adsorbents 1 to 8. The results were shown in Table 2.
Table 2

| Examples | Adsorbed Cytokine Ratio (%) | | Adsorbent Number |
| --- | --- | --- | --- |
| | IL-1 | IL-6 | |
| 11 | 37 | 38 | 1 |
| 12 | 65 | 72 | 2 |
| 13 | 61 | 87 | 3 |
| 14 | 78 | 97 | 4 |
| 15 | 42 | 42 | 5 |
| 16 | 67 | 77 | 6 |
| 17 | 39 | 40 | 7 |
| 18 | 35 | 38 | 8 |

[Examples 19 to 26]

A cylindrical column having an internal volume of 2 mL was filled with the predetermined amount of the adsorbent 1, 2, 5, 6, 7 or 8 . At 37°C for one hour, 25 mL of the blood collected with heparin (heparin concentration: 10 U/mL) from the healthy volunteer was circulated in each column at a blood flow rate of 0.65 mL/minute. The numbers of the adsorbed blood cells were calculated according to "Measurement of number of adsorbed blood cells per unit weight of adsorbent, the miniature column method" described above, and the results were shown in Table 3. Also in Table 3, the surface area, the filled volume, the fiber diameter, the adsorbing functional group, the adsorbent weight and the adsorbent number of the adsorbent in the column used in each Example are shown together.
Table 3

| Examples | Surface Area (m²) | Filled Volume (ml) | Adsorbed Blood Cells Numbers (Numbers/Adsorbent Weight (g)) | | | | Fiber Diameter (μm) | Adsorbin Functional Group | Adsorbent Weight (g) | Adsorbent Number |
|---|---|---|---|---|---|---|---|---|---|---|
| | | | Granulocytes (a) | Monocytes (b) | Lymphocytes (c) | a/2c(>1) | | | | |
| 19 | 0.8 | 2 | $5.7 \times 10^7$ | $5.5 \times 10^6$ | $8.6 \times 10^6$ | 3.3 | 4.2 | Dimethyloctylamine Group | 0.120 | 1 |
| 20 | 1.8 | 2 | $2.0 \times 10^8$ | $1.7 \times 10^7$ | $1.9 \times 10^7$ | 5.3 | 4.2 | Dimethyloctylamine Group | 0.118 | 2 |
| 21 | 0.7 | 2 | $4.7 \times 10^7$ | $2.4 \times 10^6$ | $8.2 \times 10^6$ | 2.9 | 8.5 | Dimethylhexylamine Group | 0.124 | 5 |
| 22 | 1.6 | 2 | $9.8 \times 10^8$ | $5.5 \times 10^7$ | $9.5 \times 10^7$ | 5.2 | 8.4 | Dimethylhexylamine Group | 0.128 | 6 |
| 23 | 4.9 | 2 | $1.9 \times 10^8$ | $1.9 \times 10^7$ | $3.8 \times 10^7$ | 2.5 | 4.2 | Dimethyloctylamine Group | 0.320 | 2 |
| 24 | 6.9 | 2 | $2.0 \times 10^8$ | $2.1 \times 10^7$ | $8.7 \times 10^7$ | 1.1 | 4.2 | Dimethyloctylamine Group | 0.385 | 2 |
| 25 | 0.6 | 2 | $4.7 \times 10^7$ | $7.5 \times 10^6$ | $4.6 \times 10^6$ | 5.1 | 4.2 | Dimethyloctylamine Group | 0.108 | 7 |
| 26 | 1.2 | 2 | $5.2 \times 10^7$ | $8.5 \times 10^6$ | $5.5 \times 10^6$ | 4.7 | 4.2 | Dimethylhexylamine Group | 0.112 | 8 |

[Comparative Examples 3 and 4]

A cylindrical column having the internal volume of 2 mL was filled with the predetermined amount of the adsorbent 2 and 5. At 37°C for one hour, 25 mL of the blood collected with heparin (heparin concentration: 10 U/mL) from the healthy volunteer was circulated in each column at a blood flow rate of 0.65 mL/minute. The numbers of the adsorbed blood cells were calculated according to "Measurement of number of adsorbed blood cells per unit weight of adsorbent, the miniature column method" mentioned in the above. The results obtained in the same way as in Examples 1 to 10 were shown in Table 4. Also in Table 4, the surface area, the filled volume, the fiber diameter, the adsorbing functional group, the adsorbent weight and the adsorbent number of the adsorbent in the column used in Comparative Examples 1 and 2 are shown together.

Table 4

| Comp. Ex. | Surface Area (m$^2$) | Filled Volume (ml) | Adsorbed Blood Cells Numbers (Numbers/Adsorbent Weight (g)) | | | | Fiber Diameter ($\mu$m) | Adsorbing Functional Group | Adsorbent Weight (g) | Adsorbent Number |
|---|---|---|---|---|---|---|---|---|---|---|
| | | | Granulocytes (a) | Monocytes (b) | Lymphocytes (c) | a/2c(> 1) | | | | |
| 3 | 10.3 | 2 | $2.0 \times 10^8$ | $2.2 \times 10^7$ | $1.1 \times 10^8$ | 0.9 | 4.2 | Dimethyloctylamine Group | 0.564 | 2 |
| 4 | 0.42 | 2 | $2.3 \times 10^7$ | $9.2 \times 10^5$ | $6.2 \times 10^6$ | 1.9 | 8.4 | Dimethylhexylamine Group | 0.075 | 5 |

※ "Compp. Ex."means Comparative Examples

**Claims**

1. A cell adsorption column filled with an adsorbent having a surface area of 0.5 m² or more and less than 10 m² and containing a fiber of which fiber diameter is 0.5 $\mu$m or more and 10 $\mu$m or less, wherein an adsorbent-filled volume is 100 mL or less.

2. The cell adsorption column according to claim 1, wherein a bulk density of a portion composed of said fiber of which fiber diameter is 0.5 $\mu$m or more and 10 $\mu$m or less in said adsorbent is 0.01 g/cm³ or more and 0.15 g/cm³ or less.

3. The cell adsorption column according to claim 1 or 2, wherein said fiber diameter is 3 $\mu$m or more and 10 $\mu$m or less.

4. The cell adsorption column according to any one of claims 1 to 3, wherein an amine residue is coupled to said adsorbent.

5. The cell adsorption column according to any one of claims 1 to 4, which adsorbs a cytokine.

6. The cell adsorption column according to claim 5, wherein said cytokine is at least one selected from the group consisting of interleukin-1 (IL-1), interleukin-6 (IL-6), interleukin-8 (IL-8), interleukin-10 (IL-10), tumor necrosis factor-$\alpha$ (TNF-$\alpha$), transforming growth factor-$\beta$ (TGF-$\beta$), vascular endothelial growth factor (VEGF) and immunosuppressive acidic protein (IAP).

7. The cell adsorption column according to any one of claims 1 to 6, which is used for circulating blood.

8. The cell adsorption column according to claim 7, wherein the following formulae (1) and (2) are satisfied when the blood from a human healthy volunteer is passed through the column at a blood flow rate of 30 mL/minutes for one hour:

$$2c < a \qquad\qquad (1)$$

and

$$b > 10^6 \qquad\qquad (2),$$

wherein,

a: the number of adsorbed granulocytes per unit weight of the adsorbent (number/g),
b: the number of adsorbed monocytes per unit weight of the adsorbent (number/g), and
c: the number of adsorbed lymphocytes per unit weight of the adsorbent (number/g).

9. The cell adsorption column according to any one of claims 1 to 8, wherein a pressure loss is 150 mmHg or less between the front and the rear of said column at the time of 1500 mL of blood has been treated when said blood whose hematocrit value has been adjusted to 40% using saline is passed through said column at a rate of 30 mL/minute in an extracorporeal circulation test using a healthy dog.

10. A method for adsorbing a cell, wherein said cell in a sample is adsorbed using the cell adsorption column according to any one of claims 1 to 9.

11. The method for adsorbing the cell according to claim 10, wherein said sample is blood.

12. The method for adsorbing the cell according to claim 10 or 11, wherein said cell is a leukocyte.

13. A leukocyte reduction therapy using the cell adsorption column according to any one of claims 1 to 9.

14. A therapy for ulcerative colitis, Crohn's disease or chronic rheumatoid arthritis using the cell adsorption column according to any one of claims 1 to 9.

# FIG.1

<div align="center">

**INTERNATIONAL SEARCH REPORT**

</div>

| International application No. |
|---|
| PCT/JP2007/069021 |

A.  CLASSIFICATION OF SUBJECT MATTER
*A61M1/36*(2006.01)i, *A61M1/02*(2006.01)i

According to International Patent Classification (IPC) or to both national classification and IPC

B.  FIELDS SEARCHED

Minimum documentation searched (classification system followed by classification symbols)
A61M1/36, A61M1/02

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X<br>Y | JP 2002-172163 A  (Toray Industries, Inc.),<br>18 June, 2002 (18.06.02),<br>All pages<br>(Family: none) | 1-4,7<br>5,6,8,9 |
| Y | JP 10-147518 A  (Toray Industries, Inc.),<br>02 June, 1998 (02.06.98),<br>Claims 5 to 7; Par. Nos. [0002], [0006], [0008],<br>[0013] to [0014], [0016] to [0028]<br>(Family: none) | 5,6,8,9 |
| Y | JP 2003-111834 A  (Toray Industries, Inc.),<br>15 April, 2003 (15.04.03),<br>Par. Nos. [0003], [0004], [0008], [0009],<br>[0011], [0016] to [0035]<br>(Family: none) | 5,6,8,9 |

☐  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    09 November, 2007 (09.11.07) | Date of mailing of the international search report<br>    20 November, 2007 (20.11.07) |
|---|---|
| Name and mailing address of the ISA/<br>    Japanese Patent Office | Authorized officer |
| Facsimile No. | Telephone No. |

Form PCT/ISA/210 (second sheet) (April 2007)

## INTERNATIONAL SEARCH REPORT

| International application No. |
|---|
| PCT/JP2007/069021 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 10-14
   because they relate to subject matter not required to be searched by this Authority, namely:
   Claims 10 to 14 pertain to [methods for treatment of the human body by therapy] and thus relate to a subject matter which this International Searching Authority is not required, under the provisions of the PCT Rule 39.1(iv), to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

Form PCT/ISA/210 (continuation of first sheet (2)) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO60193468 A
- JP HEI5168706 A
- JP HEI10225515 A
- JP HEI12237585 A
- JP HEI10147518 A
- JP 2002113097 A
- JP 2002172163 A